(19) Europäisches Patentamt
European Patent Office
Office européen des brevets

(11) Veröffentlichungsnummer: **0 244 641 B1**

(12) # EUROPÄISCHE PATENTSCHRIFT

(45) Veröffentlichungstag der Patentschrift: **21.08.91**

(51) Int. Cl.[5]: **C07C 217/04**, C07D 307/91, A01N 39/00, A01N 43/08

(21) Anmeldenummer: **87104969.8**

(22) Anmeldetag: **03.04.87**

(54) **1,4-Cyclohexandiamine, Verfahren zu ihrer Herstellung und ihre Verwendung als Fungizide.**

(30) Priorität: **16.04.86 DE 3612831**

(43) Veröffentlichungstag der Anmeldung:
**11.11.87 Patentblatt 87/46**

(45) Bekanntmachung des Hinweises auf die Patenterteilung:
**21.08.91 Patentblatt 91/34**

(84) Benannte Vertragsstaaten:
**AT BE CH DE ES FR GB GR IT LI NL SE**

(56) Entgegenhaltungen:
**EP-A- 0 103 277**

(73) Patentinhaber: **BASF Aktiengesellschaft**
**Carl-Bosch-Strasse 38**
**W-6700 Ludwigshafen(DE)**

(72) Erfinder: **Rentzea, Costin, Dr.**
**Richard-Kuhn-Strasse 1-3**
**W-6900 Heidelberg(DE)**
Erfinder: **Sauter, Hubert, Dr.**
**Neckarpromenade 20**
**W-6800 Mannheim 1(DE)**
Erfinder: **Goetz, Norbert, Dr.**
**Schoefferstrasse 25**
**W-6520 Worms 1(DE)**
Erfinder: **Graf, Hermann, Dr.**
**Ginsterstrasse 15**
**W-6704 Mutterstadt(DE)**
Erfinder: **Ammermann, Eberhard, Dr.**
**Sachsenstrasse 3**
**W-6700 Ludwigshafen(DE)**
Erfinder: **Pommer, Ernst-Heinrich, Dr.**
**Berliner Platz 7**
**W-6703 Limburgerhof(DE)**

**EP 0 244 641 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft neue 1,4-Cyclohexandiamine, Verfahren zu deren Herstellung, ihre Verwendung als Fungizide, fungizide Mittel, die die neuen Wirkstoffe enthalten, Verfahren zur Herstellung solcher fungiziden Mittel sowie Verfahren zur Bekämpfung von Schadpilzen mit diesen Fungiziden.

Es ist bekannt, N-Trichlormethylthio-tetrahydrophthalimid als Fungizid zu verwenden (Chemical Week, June 21, 1972, Seite 46).

Es wurde gefunden, daß neue 1,4-Cyclohexandiamine der Formel I

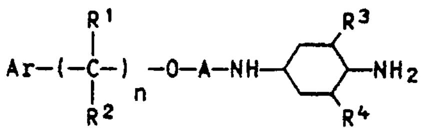

I,

worin Ar durch Halogen oder $C_1$-$C_4$-Alkyl substituiertes Dibenzofuran-3-yl, 1- und 2-Naphthyl, Phenyl oder einen durch Halogen, Trifluormethyl, $C_1$- bis $C_4$-Alkyl, $C_1$- bis $C_4$-Alkoxy, Nitro, Cyano substituierter Phenylrest bedeutet, und

$R^1$ und $R^2$ gleich oder verschieden sind und Wasserstoff, Methyl oder Ethyl bedeuten, und

$R^3$ und $R^4$ gleich oder verschieden sind und Methyl, Ethyl, n-Propyl, Isopropyl, n-Butyl, sek.-Butyl, Isobutyl, tert.-Butyl oder Isoamyl bedeuten, und

A eine Kohlenstoffkette mit 1 bis 10 C-Atomen, die durch 1 bis 3 $C_1$- bis $C_3$-Alkylreste substituiert sein kann, und

n die ganzen Zahlen 0, 1, 2, 3 und 4 bedeutet, sowie deren Salze, ausgezeichnet wirksam gegen Schadpilze sind, insbesondere gegen Phycomyceten und gute Pflanzenverträglichkeit zeigen.

Verbindungen mit Ar in der Bedeutung 7-Chlordibenzofuran-3-yl oder 4-tert.-Butyl-phenyl, n = 0, A 1,3-Propylen, 1,4-Butylen oder 1,6-Hexylen und $R^3$ und $R^4$ Isopropyl werden bevorzugt.

Die neuen Amine der Formel I enthalten ggf. chirale Zentren. Sie werden im allgemeinen als Racemate und gegebenenfalls als Diastereomerengemische erhalten. Einheitliche Diastereomere lassen sich bei einigen der neuen Verbindungen beispielsweise durch Säulenchromatographie oder aufgrund von Löslichkeitsunterschieden in reiner Form isolieren. Aus solchen gereinigten Diastereomeren kann man mit bekannten Methoden einheitliche Racemate und Enantiomere erhalten. Alle diese Verbindungen und Gemische werden von der vorliegenden Erfindung umfaßt. Für die Anwendung der neuen Amine als Fungizide sind sowohl die einheitlichen Diastereomeren bzw. Enantiomeren wie auch deren bei der Synthese anfallenden Gemische geeignet. Bevorzugt werden die letzteren verwendet.

Die Cyclohexandiamine der Formel I lassen sich herstellen, indem man

a) einen Aldehyd oder ein Keton der Formel II mit einem Amin der Formel III

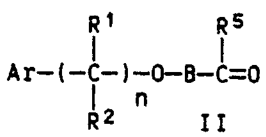

II

+

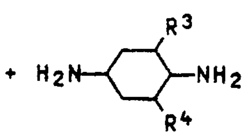

III,

in welcher $R^1$, $R^2$, $R^3$, $R^4$, Ar und n die oben genannten Bedeutungen haben und $R^3$ Wasserstoff oder $C_1$- bis $C_3$-Alkyl bedeutet und B durch den Rest -B-$CH_2$- ist A definiert wird, in Gegenwart von Ameisensäure oder Natriumcyanborhydrid oder in Gegenwart von Wasserstoff und einem Hydrierkatalysator wie Ni, Pd oder Pt umsetzt, oder

b) eine Verbindung der Formel IV mit einem Amin der Formel III

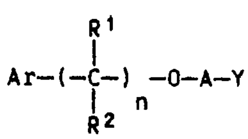

IV + III ,

in welcher $R^1$, $R^2$, Ar, A und n die oben genannten Bedeutungen haben und Y für eine nucleophil verdrängbare Abgangsgruppe steht, umsetzt und die erhaltenen Verbindungen in ihre Salze überführt.

Für den Fall, daß A die Bedeutung $-CH_2-$ hat, hat B die Bedeutung - . Ar bedeutet durch Halogen (Chlor, Brom, Fluor) oder $C_1$-$C_4$-Alkyl (Methyl) substituiertes Dibenzofuran-3-yl z.B. Dichlorbenzofuran-3-yl oder einen beispielsweise durch $C_1$-$C_4$-Alkyl (Methyl, Ethyl, tert.-Butyl) oder $C_1$-$C_4$Alkoxy (Methoxy, Ethoxy) substituierten Phenylrest.

A bedeutet insbesondere eine Alkylenkette mit 2 bis 6 C-Atomen in der Alkylenkette. $C_1$-$C_3$-Alkyl ist beispielsweise Methyl, Ethyl, Propyl.

Gemäß Verfahrensvariante a) werden Amine der Formel III mit Aldehyden oder Ketonen der Formel II z.B. in Gegenwart von Ameisensäure umgesetzt. Diese reduktive Alkylierung wird vorzugsweise in Abwesenheit eines Lösungsmittels durchgeführt. Zur Lösung des Amins in Ameisensäure wird bei Temperaturen von 0 °C bis 110 °C, vorzugsweise 50 bis 100 °C der Aldehyd oder das Keton zugetropft.

Dagegen werden die Umsetzungen der Aldehyde oder Ketone II mit Aminen der Formel III z.B. in Gegenwart von Natriumborhydrid oder Natriumcyanborhydrid in einem Lösungs- oder Verdünnungsmittel durchgeführt. Dazu eignen sich vorzugsweise Alkohole wie Methanol, Ethanol, Propanol und Isopropanol, die bis zu 25 % (Vol.%) Wasser enthalten können.

Gemäß Verfahrensvariante a) können Amine der Formel III mit Aldehyden oder Ketonen der Formel II auch in Gegenwart von Wasserstoff und einem Hydrierkatalysator alkyliert werden, beispielsweise bei einem Wasserstoffdruck von 1 bis 150 bar und einer Temperatur von 25 bis 120 °C.

Als Katalysatoren eignen sich Edelmetalle wie beispielsweise Palladium, Platin - gegebenenfalls auf einem Träger niedergeschlagen - sowie Rhodium und Raney-Nickel. Bevorzugt ist Palladium auf Kohle. Geeignete Lösungsmittel sind Alkohole wie Methanol oder Ethanol, Kohlenwasserstoffe wie Hexan, Heptan, Octan, Cyclohexan, Toluol oder Xylol. Wird eine Perhydrierung angestrebt, so wird als Katalysator Platin in Eisessig unter Zusatz von Perchlorsäure verwendet. Unter diesen Bedingungen wird der aromatische Rest durchhydriert.

Für beide Verfahrensvarianten a) und b) kommen als Lösungs- oder Verdünnungsmittel beispielsweise Halogenkohlenwasserstoffe, insbesondere Chlorkohlenwasserstoffe, z.B. Tetrachlorethylen-1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1,2,4-Trichlorbenzol; Ether, z.B. Ethylpropylether, Methyl-tert.-butylether, n-Butylethylether, Di-n-butylether, Di-isobutylether, Di-isobutylether, Diisoamylether, Diisopropylether, Anisol, Phenethol, Cyclohexylmethylether, Ethylenglykoldimethylether, Tetrahydrofuran, Dioxan, Thioanisol, $\beta,\beta,\beta'$-Dichlordiethylether; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyrnitril, Benzonitril, m-Chlorbenzonitril, aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester z.B. Ethylacetat, Acetessigester, Isobutylacetat; Amide, z.B. Formamid, Methylformamid, Dimethylformamid; Ketone, z.B. Aceton, Methylethylketon, gegebenenfalls auch Wasser und entsprechende Gemische in Betracht. Als Lösungsmittel können auch die Verbindungen der Formel III und IV im Überschuß verwendet werden. Zweckmäßig verwendet man das Lösungsmittel in einer Menge von 100 bis 2000 Gew.%, vorzugsweise von 200 bis 700 Gew.%, bezogen auf Ausgangsstoff II.

Die Verfahrensvariante b) wird beispielsweise bei 10 bis 150 °C durchgeführt.

Als anorganische oder organische Basen (Säureakzeptoren) für die Umsetzung zu Verbindungen der Formel I können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Es kommen z.B. als basische Verbindungen in Frage:

Kaliumhydroxid, Natriumhydroxid, Kaliumcarbonat, Natriumcarbonat, Lithiumhydroxid, Lithiumcarbonat, Natriumcarbonat, Kaliumcarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Tripropylamin, Tributylamin, Triisobutylamin, Tri-sec.-butylamin, Tritert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidin, N-Ethylpyrrolidion, N-Methylpiperidin, N-Ethylpiperidin, N-Methylpyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, α-Picolin, β-Picolin, γ-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N-Dimethylcyclohexylamin, 2,6-Lutidin, 2,4-Lutidin, Trifurylamin, Triethylendiamin.

Zweckmäßig verwendet man den Säureakzeptor in einem Überschuß oder Unterschuß von bis zu 20 %, bezogen auf den Ausgangsstoff IV.

Als Reaktionsbeschleuniger kommen vorzugsweise Metallhalogenide wie Natriumiodid oder Kaliumiodid in Frage.

Als nucleophil verdrängbare Abgangsgruppe Y wird beispielsweise Halogen genannt.

Zur Salzbildung mit Verbindungen der Formel I sind alle organischen und anorganischen Säuren geeignet, soweit sie pflanzenphysiologisch verträgliche Salze bilden. So sind z.B. Chloride, Bromid, Jodide, Sulfate, Phosphate, Acetate, Oxalate, Fumarate, Malonate, Alkylsulfonate und Acrylsulfonate, Dodecylbenzylsulfonate zu nennen.

Die Salze werden erhalten, indem man die entsprechende Säure mit freiem Amin der Formel I, gegebenenfalls in einem inerten Lösungsmittel zusammengibt, das Lösungsmittel abtrennt und den Rückstand gegebenenfalls umkristallisiert.

Die Ausgangsstoffe der Formel II, III und IV sind bekannt und/oder lassen sich nach an sich bekannten Methoden darstellen.

Die Ausgangsstoffe der Formel IV können leicht nach bekannten Verfahren hergestellt werden, beispielsweise durch Veretherung von Phenolen mit aliphatischen Dihalogeniden, bevorzugt in siedendem Aceton, Methylethylketon, Diethylketon oder Cyclopentanon oder in Dimethylformamid in Gegenwart von mindestens äquivalenten Mengen Natrium- oder Kaliumcarbonat (Houben-Weyl-Müller, Methoden der organischen Chemie, Band 6/3, Seiten 54 bis 59, Georg Thieme-Verlag, Stuttgart, 1965).

Alternativ können Phenoxy- oder Phenylalkoxyalkanole mit Thionylchlorid oder Phosphortribromid zu Verbindungen der Formel III umgesetzt werden (Rec. trav. chim. 76, 129 bis 145, (1957)). Dibenzofuran-3-oxyalkanole und Phenoxy-alkanole erhält man durch Umsetzung von Dibenzofuran-3-olen, von Naphtholen oder Phenolen mit aliphatischen Oxiranen oder aliphatischen, cyclischen Carbonaten nach bekannten Methoden.

Die folgenden Beispiele erläutern die Herstellung der Verbindung der Formel I:

Beispiel 1

30 g (0,15 Mol) 1,4-Diamino-2,6-diisopropylcyclohexan (Diastereomerengemisch) werden nacheinander mit 11,5 g (0,2 Mol) Calciumoxid und 23,5 g (0,1 Mol) 2-(2'-Chlorphenoxy)-ethylbromid versetzt und 10 Stunden bei 110°C gerührt. Nach Abkühlen auf 25°C wird das Gemisch mit 100 ml Diethylether versetzt, der anorganische Niederschlag abgesaugt und das Filtrat mit 80 ml 20 %iger wäßriger Natronlauge 10 Minuten gerührt. Die organische Phase wird zweimal mit je 100 ml Wasser gewaschen, getrocknet und im Vakuum fraktioniert destilliert. Man erhält 14 g 1-Amino-4-2-(2'-chlorphenoxy)-ethyl-amino-2,6-diisopropylcyclohexan vom Sdp. 187-197°C/0,1 mbar und $n_D^{24}$ 1.5279 (Verbindung Nr. 1).

Entsprechend können durch Wahl der Ausgangsstoffe und entsprechende Anpassung der Verfahrensbedingungen die nachstehend aufgelisteten Verbindungen erhalten werden:

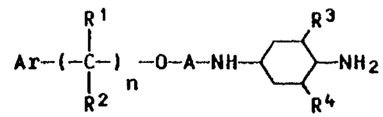

| Beispiel Nr. | Ar | 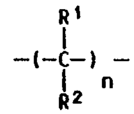 | A | $R^3$ | $R^4$ | IR-Spektrum [$cm^{-1}$] oder Phys. Konstante |
|---|---|---|---|---|---|---|
| 2 | 7-Chlordibenzofuran-3-yl | n=0 | $-(CH_2)_2-$ | $-CH_3$ | $-C(CH_3)_3-$ | Harz |
| 3 | 7-Chlordibenzofuran-3-yl | n=0 | $-(CH_2)_2-$ | $-CH(CH_3)_2$ | $-CH(CH_3)_2$ | Harz |
| 4 | 7-Chlordibenzofuran-3-yl | n=0 | $-(CH_2)_2-$ | iso-$C_4H_9$- | iso-$C_4H_9$ | Harz |
| 5 | 7-Chlordibenzofuran-3-yl | n=0 | $-(CH_2)_2-$ | sek.-$C_4H_9$- | sek.-$C_4H_9$ | Harz |
| 6 | 6-Chlordibenzofuran-3-yl | n=0 | $-(CH_2)_4-$ | $-CH(CH_3)_2$ | $-CH(CH_3)_2$ | Harz |
| 7 | 6,7-Dichlordibenzofuran-3-yl | n=0 | $-(CH_2)_4-$ | $-CH(CH_3)_2$ | $-CH(CH_3)_2$ | Harz |
| 8 | 6-$CF_3$-Dibenzofuran-3-yl | n=0 | $-(CH_2)_2-$ | $-CH(CH_3)_2$ | $-CH(CH_3)_2$ | Harz |
| 9 | 7-$CH_3$-Dibenzofuran-3-yl | n=0 | $-(CH_2)_2-$ | $-CH(CH_3)_2$ | $-CH(CH_3)_2$ | Harz |
| 10 | 7-Chlordibenzofuran-3-yl | n=0 | $-(CH_2)_3-$ | $-CH(CH_3)_2$ | $-CH(CH_3)_2$ | 2956, 2868, 1485, 1466, 1286, 1186, 1177, 1060 906, 812 |
| 11 | 7-Chlordibenzofuran-3-yl | n=0 | $-(CH_2)_4-$ | $-CH(CH_3)_2$ | $-CH(CH_3)_2$ | 2955, 1485, 1466, 1286, 1186, 1177, 1122, 1060, 908, 812 |
| 12 | 7-Chlordibenzofuran-3-yl | n=0 | $-(CH_2)_6-$ | $-CH(CH_3)_2$ | $-CH(CH_3)_2$ | 2934, 2867, 1485, 1466, 1286, 1223, 1186, 1177, 1122, 1060, 906, 812 |
| 13 | 1-Naphthyl | n=0 | $-(CH_3)_8-$ | $-CH_3$ | $-C(CH_3)_3$ | Harz |
| 14 | 2-Naphthyl | n=0 | $-(CH_2)_8-$ | $-CH(CH_3)_2$ | $-CH(CH_3)_2$ | Harz |

| Beispiel Nr. | Ar | $-(-C(R^1)(R^2)-)_n-$ | A | $R^3$ | $R^4$ | IR-Spektrum $[cm^{-1}]$ oder Phys. Konstante |
|---|---|---|---|---|---|---|
| 15 | $C_6H_5$ | n=0 | $-(CH_2)_2-$ | $-C_2H_5$ | $-CH(CH_3)_2$ | Harz |
| 16 | $C_6H_5$ | n=0 | $-(CH_2)_2-$ | $-CH(CH_3)_2$ | $-CH(CH_3)_2$ | Öl |
| 17 | $C_6H_5$ | n=0 | $-(CH_2)_4-$ | $-CH(CH_3)_2$ | $-CH(CH_3)_2$ | Öl |
| 18 | $C_6H_5$ | n=0 | $-(CH_2)_4-$ | $-CH(CH_3)_2$ | $-CH(CH_3)_2$ | Öl |
| 19 | $C_6H_5$ | $-CH_2-$ | $-(CH_2)_4-$ | $-CH(CH_3)_2$ | $-CH(CH_3)_2$ | Öl |
| 20 | $C_6H_5$ | $-(CH_2)_3-$ | $-(CH_2)_4-$ | $-CH(CH_3)_2$ | $-CH(CH_3)_2$ | Öl |
| 21 | $4-CH_3-C_6H_4-$ | n=0 | $-(CH_2)_2-$ | $-CH(CH_3)_2$ | $-CH(CH_3)_2$ | Öl |
| 22 | $4-CH_3-C_6H_4-$ | n=0 | $-(CH_2)_4-$ | $-CH(CH_3)_2$ | $-CH(CH_3)_2$ | Öl |
| 23 | $4-CH_3-C_6H_4-$ | $-CH_2-$ | $-(CH_2)_4-$ | $-CH(CH_3)_2$ | $-CH(CH_3)_2$ | Öl |
| 24 | $4-CH_3-C_6H_4-$ | $-(CH_2)_2$ | $-(CH_2)_4-$ | $-CH(CH_3)_2$ | $-CH(CH_3)_2$ | $n_D^{22}$ 1.5280, Kp. 195 - 198°C/0,2 mbar |
| 25 | $4-(CH_3)_3-C-C_6H_4-$ | n=0 | $-(CH_2)_2-$ | $-CH(CH_3)_2$ | $-CH(CH_3)_2$ | $n_D^{22}$ 1.5159, Kp. 206 - 211°C/0,3 mbar |
| 26 | $4-(CH_3)_3-C-C_6H_4-$ | n=0 | $-(CH_2)_4-$ | $-CH(CH_3)_2$ | $-CH(CH_3)_2$ | $n_D^{22}$ 1.4908, Kp. 232 - 235°C/0,5 mbar |
| 27 | $4-(CH_3)_3-C-C_6H_4-$ | n=0 | $-(CH_2)_6-$ | $-CH(CH_3)_2$ | $-CH(CH_3)_2$ | Kp. 230-234°C/0,2 mbar |
| 28 | $4-(CH_3)_3-C-C_6H_4-$ | $-CH_2-$ | $-(CH_2)_4-$ | $-CH(CH_3)_2$ | $-CH(CH_3)_2$ | Kp. 203-209°C/0,2 mbar |

| Beispiel Nr. | Ar | $-(-C(R^1)(R^2)-)_n-$ | A | $R^3$ | $R^4$ | IR-Spektrum [$cm^{-1}$] oder Phys. Konstante |
|---|---|---|---|---|---|---|
| 29 | $4-(CH_3)_3-C-C_6H_4-$ | $-CH_2-\overset{CH_3}{CH}-CH_2-$ | $-(CH_2)_4-$ | $-CH(CH_3)_2$ | $-CH(CH_3)_2$ | $n_D^{22}$ 1.5059, Kp. 248 - 252$^0$C/0,2 mbar |
| 30 | $4-(CH_3)_3-C-(C_6H_4-$ | $-CH_2-\overset{CH_3}{CH}-CH_2$ | $-(CH_2)_6-$ | $-CH(CH_3)_2$ | $-CH(CH_3)_2$ | KP. 240-249$^0$C/0,1 mbar |
| 31 | $4-ClC_6H_4-$ | n=0 | $-(CH_2)_2-$ | $-CH(CH_3)_2$ | $-CH(CH_3)_2$ | $n_D^{22}$ 1.5280, Kp. 195 - 198$^0$C/0,25 mbar |
| 32 | $4-ClC_6H_4-$ | n=0 | $-(CH_2)_3-$ | $-CH(CH_3)_2$ | $-CH(CH_3)_2$ | Kp. 202-210$^0$C/0,2 mbar |
| 33 | $4-ClC_6H_4-$ | n=0 | $-(CH_2)_4-$ | $-CH(CH_3)_2$ | $-CH(CH_3)_2$ | Kp. 225-232$^0$C/0,3 mbar |
| 34 | $4-ClC_6H_4-$ | n=0 | $-(CH_2)_5-$ | $-CH(CH_3)_2$ | $-CH(CH_3)_2$ | Kp. 218-226$^0$C/0,1 mbar |
| 35 | $4-ClC_6H_4-$ | n=0 | $-(CH_2)_6-$ | $-CH(CH_3)_2$ | $-CH(CH_3)_2$ | Kp. 228-233$^0$C/0,1 mbar |
| 36 | $4-ClC_6H_4-$ | $-CH_2-$ | $-(CH_2)_4-$ | $-CH(CH_3)_2$ | $-CH(CH_3)_2$ | Kp. 230-238$^0$C/0,2 mbar |
| 37 | $2-ClC_6H_4-$ | n=0 | $-(CH_2)_2-$ | $-CH(CH_3)_2$ | $-CH(CH_3)_2$ | $n_D^{24}$ 1.5279 |
| 38 | $2-ClC_6H_4-$ | n=0 | $-(CH_2)_3-$ | $-CH(CH_3)_2$ | $-CH(CH_3)_2$ | Kp. 190-195$^0$C/0,1 mbar |
| 39 | $2-ClC_6H_4-$ | n=0 | $-(CH_2)_4-$ | $-CH(CH_3)_2$ | $-CH(CH_3)_2$ | Kp. 198-203$^0$C/0,2 mbar |
| 40 | $3-ClC_6H_4-$ | n=0 | $-(CH_2)_3-$ | $-CH(CH_3)_2$ | $-CH(CH_3)_2$ | Kp. 202-206$^0$C/0,3 mbar |
| 41 | $3-ClC_6H_4-$ | n=0 | $-(CH_2)_4-$ | $-CH(CH_3)_2$ | $-CH(CH_3)_2$ | Kp. 209-216$^0$C/0,3 mbar |
| 42 | $2,4-Cl_2C_6H_3-$ | n=0 | $-(CH_2)_2-$ | $-CH(CH_3)_2$ | $-CH(CH_3)_2$ | $n_D^{22}$ 1.5347 |
| 43 | $2,4-Cl_2C_6H_3-$ | n=0 | $-(CH_2)_3-$ | $-CH(CH_3)_2$ | $-CH(CH_3)_2$ | Kp. 212-220$^0$C/0,3 mbar |
| 44 | $2,4-Cl_2C_6H_3-$ | $-CH_2-$ | $-(CH_2)_4-$ | $-CH(CH_3)_2$ | $-CH(CH_3)_2$ | Kp. 225-230$^0$C/0,3 mbar |

| Beispiel Nr. | Ar | $-(-C(R^1)(R^2)-)_n-$ | A | $R^3$ | $R^4$ | IR-Spektrum [$cm^{-1}$] oder Phys. Konstante |
|---|---|---|---|---|---|---|
| 45 | $3,4-Cl_2C_6H_3-$ | n=0 | $-(CH_2)_2-$ | $-CH(CH_3)_2$ | $-CH(CH_3)_2$ | $n_D^{22}$ 1.53720$^0$C/0,3 mbar |
| 46 | $3,4-Cl_2C_6H_3-$ | n=0 | $-(CH_2)_3-$ | $-CH(CH_3)_2$ | $-CH(CH_3)_2$ | Kp. 210-215$^0$C/0,2 mbar |
| 47 | $3,4-Cl_2C_6H_3-$ | n=0 | $-(CH_2)_5-$ | $-CH(CH_3)_2$ | $-CH(CH_3)_2$ | Kp. 230-232$^0$C/0,4 mbar |
| 48 | $2,6-Cl_2C_6H_3-$ | n=0 | $-(CH_2)_2-$ | $-CH(CH_3)_2$ | $-CH(CH_3)_2$ | Kp. 202-208$^0$C/0,2 mbar |
| 49 | $2,6-Cl_2C_6H_3-$ | n=0 | $-(CH_2)_4-$ | $-CH(CH_3)_2$ | $-CH(CH_3)_2$ | Kp. 228-235$^0$C/0,1 mbar |
| 50 | $2,6-Cl_2C_6H_3-$ | n=0 | $-(CH_2)_6-$ | $-CH(CH_3)_2$ | $-CH(CH_3)_2$ | Kp. 246-250$^0$C/0,2 mbar |
| 51 | $2-FC_6H_4-$ | n=0 | $-(CH_2)_2-$ | $-CH(CH_3)_2$ | $-CH(CH_3)_2$ | Kp. 184-189$^0$C/0,2 mbar |
| 52 | $2-FC_6H_4-$ | n=0 | $-(CH_2)_4-$ | $-CH(CH_3)_2$ | $-CH(CH_3)_2$ | Kp. 186-191$^0$C/0,2 mbar |
| 53 | $2-FC_6H_4-$ | n=0 | $-(CH_2)_6-$ | $-CH(CH_3)_2$ | $-CH(CH_3)_2$ | Kp. 198-204$^0$C/0,2 mbar |
| 54 | $4-FC_6H_4-$ | n=0 | $-(CH_2)_4-$ | $-CH(CH_3)_2$ | $-CH(CH_3)_2$ | Kp. 189-194$^0$C/0,4 mbar |
| 55 | $4-BrC_6H_4-$ | n=0 | $-(CH_2)_4-$ | $-CH(CH_3)_2$ | $-CH(CH_3)_2$ | Kp. 209-212$^0$C/0,1 mbar |
| 56 | $2,4,6-Cl_3C_6H_2-$ | n=0 | $-(CH_2)_2-$ | $-CH(CH_3)_2$ | $-CH(CH_3)_2$ | Öl |
| 57 | $2,4,6-Cl_3C_6H_2-$ | n=0 | $-(CH_2)_2-$ | $-CH(CH_3)_2$ | $-CH(CH_3)_2$ | Öl |
| 58 | $2,4,6-Cl_3C_6H_2-$ | n=0 | $-(CH_2)_4-$ | $-CH(CH_3)_2$ | $-CH(CH_3)_2$ | Öl |
| 59 | $2,4,6-Cl_3C_6H_2-$ | n=0 | $-(CH_2)_{10}-$ | $-CH(CH_3)_2$ | $-CH(CH_3)_2$ | Öl |
| 60 | $2,4-(CH_3)_2C_6H_3-$ | n=0 | $-(CH_2)_2-$ | $-CH(CH_3)_2$ | $-CH(CH_3)_2$ | Kp. 200-204$^0$C/0,2 mbar |
| 61 | $2,4-(CH_3)_2C_6H_3-$ | n=0 | $-(CH_2)_4-$ | $-CH(CH_3)_2$ | $-CH(CH_3)_2$ | Kp. 231-236$^0$C/0,3 mbar |
| 62 | $2,4-(CH_3)_2C_6H_3-$ | n=0 | $-(CH_2)_4-$ | $-CH(CH_3)_2$ | $-CH(CH_3)_2$ | Öl |
| 63 | $2,4,6-(CH_3)_3C_6H_2-$ | n=0 | $-(CH_2)_2-$ | $-CH(CH_3)_2$ | $-CH(CH_3)_2$ | Kp. 220-223$^0$C/0,2 mbar |

| Beispiel Nr. | Ar | $-(CR^1R^2)_n-$ | A | $R^3$ | $R^4$ | IR-Spektrum [$cm^{-1}$] oder Phys. Konstante |
|---|---|---|---|---|---|---|
| 64 | $2,4,6-(CH_3)_3C_6H_2-$ | n=0 | $-(CH_2)_4-$ | $-CH(CH_3)_2$ | $-CH(CH_3)_2$ | Kp. 251-260°C/0,3 mbar |
| 65 | $4-(CF_3)C_6H_4-$ | n=0 | $-(CH_2)_2-$ | $-CH(CH_3)_2$ | $-CH(CH_3)_2$ | Kp. 197-201°C/0,2 mbar |
| 66 | $4-(CF_3)C_6H_4-$ | n=0 | $-(CH_2)_4-$ | $-CH(CH_3)_2$ | $-CH(CH_3)_2$ | Kp. 196-205°C/0,1 mbar |
| 67 | $4-(CF_3)C_6H_4-$ | $-CH_2-$ | $-(CH_2)_4-$ | $-CH(CH_3)_2$ | $-CH(CH_3)_2$ | Kp. 209-211°C/0,2 mbar |
| 68 | $4-O_2N-C_6H_4-$ | n=0 | $-(CH_2)_4-$ | $-CH(CH_3)_2$ | $-CH(CH_3)_2$ | Öl |
| 69 | $4-O_2N-C_6H_4-$ | n=0 | $-(CH_2)_6-$ | $-CH(CH_3)_2$ | $-CH(CH_3)_2$ | Öl |
| 70 | $4-NC-C_6H_4-$ | n=0 | $-(CH_2)_2-$ | $-CH(CH_3)_2$ | $-CH(CH_3)_2$ | Öl |

$B_1$ und $B_2$ wurden entsprechend Schema 1, d.h. wie in DE 26 54 890 beschrieben, hergestellt.

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle,
Ustilago-Arten an Getreide und Zuckerrohr,
Venturia inaequalis (Schorf) an Äpfeln,
Septoria nodorum an Weizen,
Helminthosporium teres an Gerste,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Alternaria solani an Kartoffeln, Tomaten,
Plasmopara viticola an Reben sowie
Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden. Bei der Anwendung der Wirkstoffe im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht. Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 10 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 11 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 12 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 25 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 26 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 29 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 31 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 42 werden mit 10 Gew.-Teilen Natriumsalz eines Phenolsulfonsäure-harnstoff-formaldehyd-Kondensates, 2 Gew.-Teilen kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 45 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,

2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalmid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
1-(4-Phenylphenoxy)-3,3-dimethyl-1-(1H)-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,

2,4'-Difluor-α-(1H-1,2,4-triazolyl-1-methyl)-benzhydrylalkohol
N-(3-Chlor, 2,6-dinitro, 4-trifluormethylphenyl-5-trifluormethyl,
3-chlor, 2-aminopyridin,
1-((bis(4-Fluorphenyl)methylsilyl)-methyl-1H-1,2,4-triazol.

Für die folgenden Versuche wurde als bekanntes Vergleichsmittel der Wirkstoff N-Trichlormethylthio-tetrahydrophthalimid verwendet.

Anwendungsbeispiel 1
Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24 °C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß die Verbindungen 10, 11, 12, 25, 26, 29, 31, 42 und 45 bei der Anwendung als 0,05 %ige Spritzbrühe eine bessere fungizide Wirkung zeigen (97 %) als das bekannte Vergleichsmittel (70 %).

Anwendungsbeispiel 2
Wirksamkeit gegen Weizenmehltau

Blätter von in Töpfen gewachsenen Weizenkeimlingen der Sorte Frühgold werden mit wäßriger Spritzbrühe, die 80 % Wirkstoff und 20 % Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22 °C und 75 bis 80 % relativer Luftfeuchtigkeit aufgestellt. Nach 7 Tagen wird das Ausmaß der Mehltauentwicklung ermittelt.

Das Ergebnis des Versuches zeigt, daß die Verbindungen 10, 11, 12 und 26 bei der Anwendung als 0,025 %ige Spritzbrühe eine sehr gute fungizide Wirkung zeigen (97 %).

Anwendungsbeispiel 3
Wirksamkeit gegen Plasmopara viticola

Blätter von Topfreben der Sorte "Müller-Thurgau" werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 8 Tage im Gewächshaus aufgestellt. Erst dann werden die Blätter mit einer Zoosporenaufschwemmung von Plasmorpara viticola (Rebenperonospora) infiziert. Danach werden die Reben zunächst für 48 Stunden in einer wasserdampfgesättigten Kammer bei 24 °C und anschließend für 5 Tage in einem Gewächshaus mit Temperaturen zwischen 20 und 30 °C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten.

Das Ergebnis des Versuches zeigt, daß die Verbindungen 10, 11, 24, 25, 26, 31, 42 und 45 bei der Anwendung als 0,05 %ige Spritzbrühe eine sehr gute fungizide Wirkung zeigen (97 %).

**Patentansprüche**

**1.** 1 , 4-Cyclohexandiamine der Formel I

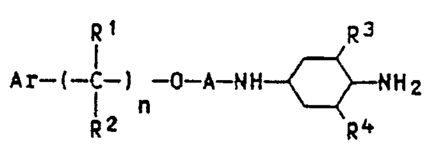

I,

worin Ar durch Halogen oder $C_1$- bis $C_4$-Alkyl substituiertes Dibenzofuran3-yl, 1- und 2-Naphthyl, Phenyl oder einen durch Halogen, Trifluormethyl, $C_1$- bis $C_4$-Alkyl, $C_1$- bis $C_4$-Alkoxy, Nitro, Cyano substituierten Phenylrest bedeutet, und $R^1$ und $R^2$ gleich oder verschieden sind und Wasserstoff, Methyl oder Ethyl bedeuten, und
$R^3$ und $R^4$ gleich oder verschieden sind und Methyl, Ethyl, n-Propyl, Isopropyl, n-Butyl, sek. -Butyl, Isobutyl, tert. -Butyl oder Isoamyl bedeuten, und
A eine Kohlenstoffkette mit 1 bis 10 C-Atomen, die durch 1 bis 3 $C_1$- bis $C_3$-Alkylreste substituiert sein kann, und
n die ganzen Zahlen O, 1, 2, 3 und 4 bedeutet, sowie deren Salze.

2. 1,4-Cyclohexandiamin gemäß Anspruch 1, dadurch gekennzeichnet, daß Ar 7-Chlordibenzofuran-3-yl, n=0, A 1 ,3-Propylen, 1,4-Butylen oder 1,6-Hexylen und $R^3$ und $R^4$ Isopropyl bedeuten.

3. 1,4-Cyclohexandiamin gemäß Anspruch 1, dadurch gekennzeichnet, daß Ar 4-tert.-Butyl-phenyl, n=O, A 1,4-Butylen und $R^3$ und $R^4$ Isopropyl bedeuten.

4. Fungizide Mittel, enthaltend ein 1,4-Cyclohexandiamin der Formel I

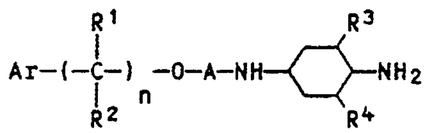

I,

worin Ar durch Halogen oder $C_1$- bis $C_4$-Alkyl substituiertes Dibenzofuran-3-yl, 1- und 2-Naphthyl, Phenyl oder einen durch Halogen, Trifluormethyl, $C_1$- bis $C_4$-Alkyl, $C_1$- bis $C_4$-Alkoxy, Nitro, Cyano substituierten Phenylrest bedeutet, und $R^1$ und $R^2$ gleich oder verschieden sind und Wasserstoff, Methyl oder Ethyl bedeuten, und
$R^3$ und $R^4$ gleich oder verschieden sind und Methyl, Ethyl, n-Propyl, Isopropyl, n-Butyl, sek. -Butyl, Isobutyl, tert. -Butyl oder Isoamyl bedeuten, und
A eine Kohlenstoffkette mit 1 bis 10 C-Atomen, die durch 1 bis 3 $C_1$- bis $C_3$-Alkylreste substituiert sein kann, und
n die ganzen Zahlen 0, 1, 2, 3 und 4 bedeutet, oder deren Salze und einen festen oder flüssigen Trägerstoff.

5. Verfahren zur Herstellung von Fungiziden, dadurch gekennzeichnet, daß man eine Verbindung der Formel I gemäß Anspruch 1 mit festen oder flüssigen Trägerstoffen mischt.

6. Verfahren zur Bekämpfung von Fungi, dadurch gekennzeichnet, daß man ein 1,4-Cyclohexandiamin der Formel I

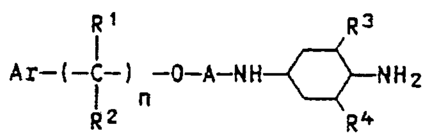

I,

worin Ar durch Halogen oder $C_1$- bis $C_4$-Alkyl substituiertes Dibenzofuran-3-yl, 1- und 2-Naphthyl, Phenyl oder einen durch Halogen, Trifluormethyl, $C_1$- bis $C_4$-Alkyl, $C_1$- bis $C_4$-Alkoxy, Nitro, Cyano substituierten Phenylrest bedeutet, und $R^1$ und $R^2$ gleich oder verschieden sind und Wasserstoff, Methyl oder Ethyl bedeuten, und
$R^3$ und $R^4$ gleich oder verschieden sind und Methyl, Ethyl, n-Propyl, Isopropyl, n-Butyl, sek. -Butyl, Isobutyl, tert. -Butyl oder Isoamyl bedeuten, und
A eine Kohlenstoffkette mit 1 bis 10 C-Atomen, die durch 1 bis 3 $C_1$- bis $C_3$-Alkylreste substituiert sein kann, und
n die ganzen Zahlen 0, 1, 2, 3 und 4 bedeutet, oder dessen Salz auf die Pilze oder durch Pilzbefall

bedrohte Flächen, Pflanzen oder Saatgüter einwirken läßt.

**7.** Verfahren zur Herstellung eines 1,4-Cyclohexandiamins der Formel I

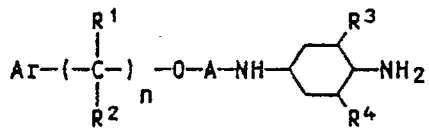

I,

worin Ar durch Halogen oder $C_1$- bis $C_4$-Alkyl substituiertes Dibenzofuran-3-yl, 1- und 2-Naphthyl, Phenyl oder einen durch Halogen, Trifluormethyl, $C_1$- bis $C_4$-Alkyl, $C_1$- bis $C_4$-Alkoxy, Nitro, Cyano substituierten Phenylrest bedeutet, und $R^1$ und $R^2$ gleich oder verschieden sind und Wasserstoff, Methyl oder Ethyl bedeuten, und
$R^3$ und $R^4$ gleich oder verschieden sind und Methyl, Ethyl, n-Propyl, Isopropyl, n-Butyl, sek.-Butyl, Isobutyl, tert.-Butyl oder Isoamyl bedeuten, und
A eine Kohlenstoffkette mit 1 bis 10 C-Atomen, die durch 1 bis 3 $C_1$- bis $C_3$-Alkylreste substituiert sein kann, und
n die ganzen Zahlen 0, 1, 2, 3 und 4 bedeutet,
dadurch gekennzeichnet, daß man
a) einen Aldehyd oder ein Keton der Formel II mit einem Amin der Formel III

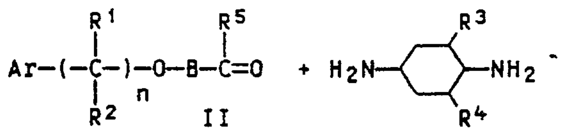

III,

in welcher $R^1$, $R^2$, $R^3$, $R^4$, Ar und n die oben genannten Bedeutungen haben und $R^3$ Wasserstoff oder $C_1$- bis $C_3$-Alkyl bedeutet und B durch den Rest -B-$CH_2$- ist A definiert wird, in Gegenwart von Ameisensäure oder Natriumcyanborhydrid oder in Gegenwart von Wasserstoff und einem Hydrierkatalysator umsetzt, oder
b) eine Verbindung der Formel IV mit einem Amin der Formel III

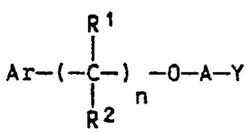

IV + III ,

in welcher $R^1$ , $R^2$, Ar, A und n die oben genannten Bedeutungen haben und Y für eine nucleophil verdrängbare Abgangsgruppe steht, umsetzt und die so erhaltenen Verbindungen gegebenenfalls in ihre Salze überführt.

## Claims

**1.** A 1,4-cyclohexanediamine of the formula I

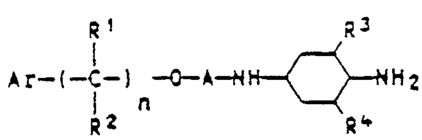

I

where Ar is dibenzofuran-3-yl which is substituted by halogen or by $C_1$-$C_4$-alkyl, or is 1- or 2-naphthyl or is phenyl which is unsubstituted or substituted by halogen, trifluoromethyl, $C_1$-$C_4$-alkyl, $C_1$-$C_4$-alkoxy, nitro or cyano, $R^1$ and $R^2$ are identical or different and are each hydrogen, methyl or ethyl, $R^3$ and $R^4$ are identical or different and are each methyl, ethyl, n-propyl, isopropyl, n-butyl, sec-butyl, isobutyl, tert-butyl or isoamyl, A is a hydrocarbon chain of 1 to 10 carbon atoms which may be substituted by 1 to 3 $C_1$-$C_3$-alkyl radicals and n is one of the integers 0, 1, 2, 3 and 4, and its salts.

2. A 1,4-cyclohexanediamine as claimed in claim 1, wherein Ar is 7-chlorodibenzofuran-3-yl, n is 0, A is 1,3-propylene, 1,4-butylene or 1,6-hexylene and $R^3$ and $R^4$ are each isopropyl.

3. A 1,4-cyclohexanediamine as claimed in claim 1, wherein Ar is 4-tert-butylphenyl, n is 0, A is 1,4-butylene and $R^3$ and $R^4$ are each isopropyl.

4. A fungicide containing a 1,4-cyclohexanediamine of the formula I

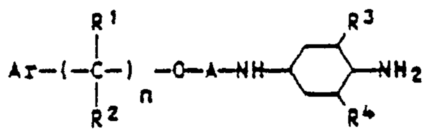

I

where Ar is dibenzofuran-3-yl which is substituted by halogen or by $C_1$-$C_4$-alkyl, or is 1- or 2-naphthyl or is phenyl which is unsubstituted or substituted by halogen, trifluoromethyl, $C_1$-$C_4$-alkyl, $C_1$-$C_4$-alkoxy, nitro or cyano, $R^1$ and $R^2$ are identical or different and are each hydrogen, methyl or ethyl, $R^3$ and $R^4$ are identical or different and are each methyl, ethyl, n-propyl, isopropyl, n-butyl, sec-butyl, isobutyl, tert-butyl or isoamyl, A is a hydrocarbon chain of 1 to 10 carbon atoms which may be substituted by 1 to 3 $C_1$-$C_3$-alkyl radicals and n is one of the integers 0, 1, 2, 3 and 4, or its salts and a solid or liquid carrier.

5. A process for the preparation of a fungicide, wherein a compound of the formula I as claimed in claim 1 is mixed with a solid or liquid carrier.

6. A method for controlling fungi, wherein a 1,4-cyclohexanediamine of the formula I

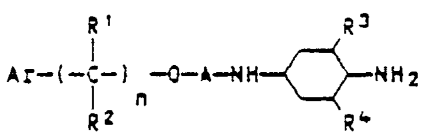

I

where Ar is dibenzofuran-3-yl which is substituted by halogen or by $C_1$-$C_4$-alkyl, or is 1- or 2-naphthyl or is phenyl which is unsubstituted or substituted by halogen, trifluoromethyl, $C_1$-$C_4$-alkyl, $C_1$-$C_4$-alkoxy, nitro or cyano, $R^1$ and $R^2$ are identical or different and are each hydrogen, methyl or ethyl, $R^3$ and $R^4$ are identical or different and are each methyl, ethyl, n-propyl, isopropyl, n-butyl, sec-butyl, isobutyl, tert-butyl or isoamyl, A is a hydrocarbon chain of 1 to 10 carbon atoms which may be substituted by 1 to 3 $C_1$-$C_3$-alkyl radicals and n is one of the integers 0, 1, 2, 3 and 4, or its salt is allowed to act on the fungi or on surfaces, plants or seeds threatened by fungal attack.

7. A process for the preparation of 1,4-cyclohexanediamine of the formula I

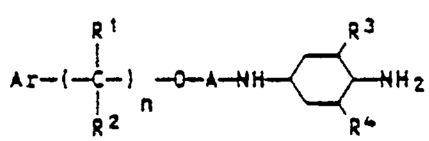

I

where Ar is dibenzofuran-3-yl which is substituted by halogen or by $C_1$-$C_4$-alkyl, or is 1- or 2-naphthyl or is phenyl which is unsubstituted or substituted by halogen, trifluoromethyl, $C_1$-$C_4$-alkyl, $C_1$-$C_4$-alkoxy, nitro or cyano, $R^1$ and $R^2$ are identical or different and are each hydrogen, methyl or ethyl, $R^3$ and $R^4$ are identical or different and are each methyl, ethyl, n-propyl, isopropyl, n-butyl, sec-butyl, isobutyl, tert-butyl or isoamyl, A is a hydrocarbon chain of 1 to 10 carbon atoms which may be substituted by 1 to 3 $C_1$-$C_3$-alkyl radicals and n is one of the integers 0, 1, 2, 3 and 4, wherein

a) an aldehyde or a ketone of the formula II is reacted with an amine of the formula III

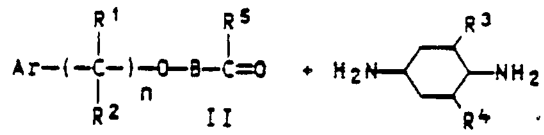

II III

where $R^1$, $R^2$, $R^3$, $R^4$, Ar and n have the abovementioned meanings and $R^3$ is hydrogen or $C_1$-$C_3$-alkyl and B is defined so that the radical -B-$CH_2$- is A, in the presence of formic acid or sodium cyanoborohydride or in the presence of hydrogen and a hydrogenation catalyst, or

b) a compound of the formula IV is reacted with an amine of the formula III

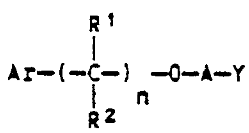

IV + III

where $R^1$, $R^2$, Ar, A and n have the abovementioned meanings and Y is a nucleophilically displaceable leaving group, and the resulting compound is, if required, converted into its salts.

## Revendications

**1.** 1,4-cyclohexane-diamines de formule I

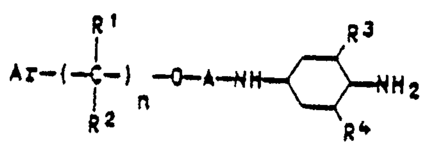

I,

dans laquelle Ar représente un groupe dibenzofuran-3-yle, 1- ou 2-naphtyle substitué par des halogènes ou des groupes alkyle en C 1-C 4, un groupe phényle ou un groupe phényle substitué par des halogènes, des groupes trifluorométhyle, alkyle en C 1-C 4, alcoxy en C 1-C 4, nitro, cyano, et $R^1$ et $R^2$, ayant des significations identiques ou différentes, représentent l'hydrogène, des groupes méthyle ou éthyle, et

$R^3$ et $R^4$, ayant des significations identiques ou différentes, représentent des groupes méthyle, éthyle, n-propyle, isopropyle, n-butyle, sec-butyle, iso-butyle, tert-butyle et isoamyle, et

A représente une chaîne carbonée contenant 1 à 10 atomes de carbone et qui peut être substituée par un à trois groupes alkyle en C 1-C 3, et

n est égal à 0; 1; 2; 3 ou 4, et leurs sels.

**2.** 1,4-cyclohexane-diamine selon la revendication 1, caractérisée en ce que Ar représente le groupe 7-chloro-dibenzofuran-3-yle, n = 0, A représente une chaîne 1,3-propylène, 1,4-butylène ou 1,6-hexylène et $R^3$ et $R^4$ représentent des groupes isopropyle.

**3.** 1,4-cyclohexane-diamine selon la revendication 1, caractérisée en ce que Ar représente un groupe 4-tert-butylphényle, n = 0, A représente une chaîne 1,4-butylène et $R^3$ et $R^4$ des groupes isopropyle.

4. Produits fongicides contenant une 1,4-cyclohexane-diamine de formule I

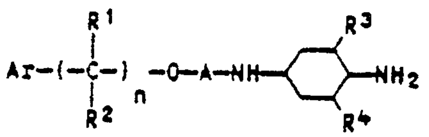

I,

dans laquelle Ar représente un groupe dibenzofuran-3-yle, 1- ou 2-naphtyle substitué par des halogènes ou des groupes alkyle en C 1-C 4, un groupe phényle ou un groupe phényle substitué par des halogènes, des groupes trifluorométhyle, alkyle en C 1-C 4, alcoxy en C 1-C 4, nitro, cyano, et $R^1$ et $R^2$, ayant des significations identiques ou différentes, représentent l'hydrogène, des groupes méthyle ou éthyle, et

- $R^3$ et $R^4$, ayant des significations identiques ou différentes, représentent des groupes méthyle, éthyle, n-propyle, isopropyle, n-butyle, sec-butyle, iso-butyle, tert-butyle ou isoamyle, et
- A représente une chaîne carbonée contenant 1 à 10 atomes de carbone et qui peut être substituée par un à trois groupes alkyle en C 1-C 3, et
- n est égal à 0, 1, 2, 3 ou 4, ou leurs sels, et

un véhicule solide ou liquide.

5. Procédé de préparation de produits fongicides, caractérisé en ce que l'on mélange un composé de formule I de la revendication 1 avec des véhicules solides ou liquides.

6. Procédé pour combattre les mycètes, caractérisé en ce que l'on fait agir sur les mycètes ou les surfaces, les végétaux ou les semences menacés d'une attaque par les mycètes, une 1,4-cyclohexane-diamine de formule I

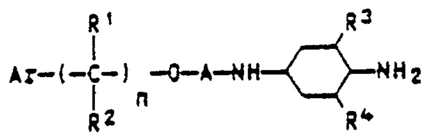

I,

dans laquelle Ar représente un groupe dibenzofuran-3-yle, 1- ou 2-naphtyle substitué par des halogènes ou des groupes alkyle en C 1-C 4, un groupe phényle ou un groupe phényle substitué par des halogènes, des groupes trifluorométhyle, alkyle en C 1-C 4, alcoxy en C 1-C 4, nitro, cyano, et $R^1$ et $R^2$, ayant des significations identiques ou différentes, représentent l'hydrogène, des groupes méthyle ou éthyle, et

- $R^3$ et $R^4$, ayant des significations identiques ou différentes, représentent des groupes méthyle, éthyle, n-propyle, isopropyle, n-butyle, sec-butyle, iso-butyle, tert-butyle ou isoamyle, et
- A représente une chaîne carbonée contenant de 1 à 10 atomes de carbone et qui peut être substituée par un à trois groupes alkyle en C 1-C 3, et
- n est égal à 0, 1, 2, 3 ou 4, ou un sel de ce composé.

7. Procédé de préparation d'une 1,4-cyclohexane-diamine de formule I

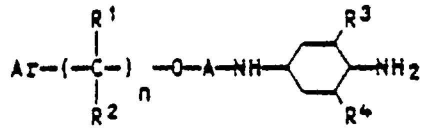

I,

dans laquelle Ar représente un groupe dibenzofuran-3-yle, 1- ou 2-naphtyle substitué par des halogènes ou des groupes alkyle en C 1-C 4, un groupe phényle ou un groupe phényle substitué par des halogènes, des groupes trifluorométhyle, alkyle en C 1-C 4, alcoxy en C 1-C 4, nitro, cyano et $R^1$ et $R^2$, ayant des significations identiques ou différentes, représentent l'hydrogène, des groupes méthyle ou éthyle, et

- $R^3$ et $R^4$, ayant des significations identiques ou différentes, représentent des groupes méthyle, éthyle, n-propyle, isopropyle, n-butyle, sec-butyle, iso-butyle, tert-butyle ou isoamyle, et
- A représente une chaîne carbonée contenant 1 à 10 atomes de carbone et qui peut être substituée par un à trois groupes alkyle en C 1-C 3, et
- n est égal à 0, 1, 2, 3 ou 4,

caractérisé en ce que :

a) on fait réagir un aldéhyde ou une cétone de formule II avec une amine de formule III

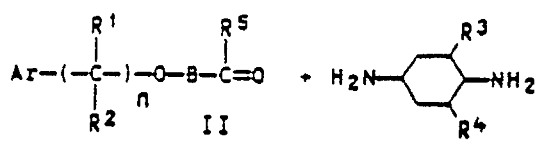

II + III,

dans lesquelles $R^1$, $R^2$, $R^3$, $R^4$, Ar et n ont les significations indiquées ci-dessus, et $R^3$ représente l'hydrogène ou un groupe alkyle en C 1-C 3 et B est défini en ce que A = $-B-CH_2-$, en présence d'acide formique ou de cyanoborohydrure de sodium ou en présence d'hydrogène et d'un catalyseur d'hydrogénation, ou bien

b) on fait réagir un composé de formule IV avec une amine de formule III

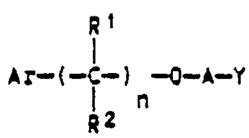

IV + III,

dans lesquelles $R^1$, $R^2$, Ar, A et n ont les significations indiquées ci-dessus, et Y représente un groupe éliminable, déplaçable par nucléophilie, et le cas échéant on convertit les composés obtenus en leurs sels.